# EUROPEAN PATENT APPLICATION

(11) **EP 3 470 998 A1**
(43) Date of publication of application: **17.04.2019**
(21) Application number: 17833441.3
(22) Date of filing: 14.07.2017
(51) Int. Cl.: G06F 19/20

(54) **DATA PROCESSING METHOD AND APPARATUS**

(30) Priority: 27.07.2016 CN 201610601815
(71) Applicant: Huawei Technologies Co., Ltd., Longgang District Shenzhen, Guangdong 518129 (CN)
(72) Inventor: LIU, Chuanjian, Shenzhen Guangdong 518129 (CN); DENG, Liqun, Shenzhen Guangdong 518129 (CN); HUANG, Guowei, Shenzhen Guangdong 518129 (CN)
(74) Representative: Isarpatent
(86) International application number: PCT/CN2017/092981
(87) International publication number: WO 2018/019138

(57) **Abstract**

Embodiments of the present invention provide a data processing method, applied to a data processing system, where the data processing system includes a reference sample and a first sample set. The method includes: traversing all sample fragments in the first sample set, collecting statistics about a first statistic of each basic element that is in the reference sample and that is included in the sample fragments, and determining that a position, in the reference sample, of a basic element whose first statistic is less than a first threshold is a spacing position; dividing the reference sample into at least two reference sub-samples, where a division point for the division includes: a spacing position that is not adjacent to another spacing position, and any one of at least two adjacent spacing positions; traversing all the sample fragments in the first sample set, and collecting statistics about a second statistic of each reference sub-sample that is of the reference sample and that includes the sample fragments; and combining, when a sum of second statistics of any adjacent reference sub-samples is less than a second threshold, the adjacent reference sub-samples.

## Description

This application claims priority to Chinese Patent Application No. 201610601815.4, filed with the Chinese Patent Office on July 27, 2016 and entitled "DATA PROCESSING METHOD AND APPARATUS", which is incorporated herein by reference in its entirety.

### TECHNICAL FIELD

This application relates to the data processing field, and in particular, to a method and an apparatus for segmenting and processing deoxyribonucleic acid sequencing data.

### BACKGROUND

Deoxyribonucleic acid (Deoxyribonucleic Acid, DNA) is a long-chain polymer and includes four types of deoxyribonucleotides. A deoxyribonucleotide includes a deoxyribose sugar, a phosphate group, and a nitrogenous base. The deoxyribose sugar and the phosphate group are connected by using an ester bond, to form an outer long-chain skeleton. Each deoxyribose sugar molecule is internally connected to one of four types of nitrogenous bases. The nitrogenous bases are sorted along a long DNA strand to form a sequence, and are bases for synthesis of an amino acid sequence of protein. The nitrogenous bases forming the deoxyribonucleic acid are adenine, thymine, cytosine, and guanine. DNA is of a double-stranded structure, that is, a nitrogenous base on one strand appears in pair with a nitrogenous base at a corresponding position on the other strand. A nitrogenous base is usually used as a length unit of DNA. In a computer system, the four types of nitrogenous bases are respectively represented as characters A, T, C, and G, and each character occupies one byte. Therefore, a byte is used as a length unit of a nitrogenous base sequence.

With advancement of DNA sequencing technologies, genetic analysis has become an important means for detection and targeted treatment of inherited/mutative diseases. The genetic analysis includes three necessary phases: DNA sequencing, DNA sequence assembly and variant calling, and gene annotation and analysis.

DNA sequencing is a process of accurately determining a nucleotide sorting sequence in a DNA molecule. Because a nucleotide type is mainly determined by a nitrogenous base, essentially, only a nitrogenous base sorting sequence needs to be determined for sequencing. To improve a sequencing speed, a to-be-sequenced genome is divided into fragments having a length of tens to hundreds of nitrogenous bases, and then a sequencer simultaneously sequences tens to millions of fragments. A sequenced DNA fragment having the length of tens to hundreds of nitrogenous bases is referred to as a read (Read). In another aspect, to improve sequencing accuracy and coverage (Coverage), repeated sequencing is usually performed on a target region of the to-be-sequenced genome during high throughput sequencing, so as to increase a sequencing depth (Sequence Depth). The coverage is a ratio of a sequence obtained by means of sequencing to the target region. Due to existence of a complex structure such as repeated sequences in a genome, a sequence finally obtained by means of assembly during sequencing usually cannot cover all regions. A region that is not covered is referred to as a gap (Gap). The sequencing depth is a ratio of a total quantity of nitrogenous bases obtained by means of sequencing to a size of the to-be-sequenced genome.

DNA sequence assembly and variant calling is a process of assembling nitrogenous base sequence reads output by the sequencer into a DNA sequence by using a computer method, and finding a variant nitrogenous base locus by comparing the DNA sequence with a reference sequence of the to-be-sequenced genome. Original coordinates of the reads in the to-be-sequenced genome can be determined only by mapping (Mapping) the reads to the reference sequence. Because human DNA has many repeated nitrogenous base sequences, some reads may be mapped to multiple positions. A SAM (Sequence Alignment/Map) format is a general comparison format and is used to store comparison information from a read to the reference sequence. In a SAM file, each row other than an annotation row includes one read and comparison information of the read, for example, a number of a chromosome to which the read is mapped and the first start position of the chromosome to which the read is mapped. The information is the coordinates of the read in the reference sequence. Data records in the SAM file are referred to as SAM data (SAM Records).

Gene annotation and analysis is a process of analyzing a sequencing result, recognizing a gene and a function of the gene, and mining a relationship between a variant and a related disease by using a bioinformatics method and with reference to proteomics and transcriptomics.

In the foregoing three essential phases of the genetic analysis, DNA sequence assembly and variant calling is a part that relates to less of the biological field and that requires a large quantity of computing overheads. Actually, due to many possible factors such as different sequencing depths of the DNA sequencing in chromosome regions and uneven distribution of processing results of sequenced data after several steps, in the DNA sequence assembly and variant calling part, computing resources usually cannot be properly allocated to analyze a result of the DNA sequencing part.

### SUMMARY

Embodiments of the present invention provide a data processing method and apparatus, so as to properly improve a degree of execution parallelism of to-be-processed data, and improve a data processing speed and data processing efficiency.

To achieve the foregoing objective, the following technical solutions are used in the embodiments of the present invention:

According to a first aspect, an embodiment of the present invention provides a data processing method, applied to a data processing system. The data processing system includes a reference sample and a first sample set, the reference sample includes at least two basic elements sorted according to preset order, the first sample set includes at least one sample fragment, and the sample fragment includes at least one basic element captured from the reference sample. The method includes: traversing all the sample fragments in the first sample set, collecting statistics about a first statistic of each basic element that is in the reference sample and that is included in the sample fragments, and determining that a position, in the reference sample, of a basic element whose first statistic is less than a first threshold is a spacing position; dividing the reference sample into at least two reference sub-samples, where a division point for the division includes: a spacing position that is not adjacent to another spacing position, and any one of at least two adjacent spacing positions; traversing all the sample fragments in the first sample set, and collecting statistics about a second statistic of each reference sub-sample that is of the reference sample and that includes the sample fragments; and combining, when a sum of second statistics of any adjacent reference sub-samples is less than a second threshold, the adjacent reference sub-samples.

In a first feasible implementation of the first aspect, the collecting statistics about a first statistic of each basic element that is in the reference sample and that is included in the sample fragments includes: traversing all the basic elements in the reference sample, and when a basic element is included in the sample fragment, adding 1 to a first statistic of the basic element.

In another feasible implementation of the first aspect, the dividing the reference sample into at least two reference sub-samples includes: traversing all the basic elements in the reference sample, and when a first statistic of a basic element is less than the first threshold, determining that a position of the basic element in the reference sample is a spacing position; determining the division point according to the spacing position; and dividing the reference sample into the at least two reference sub-samples according to the division point.

In another feasible implementation of the first aspect, the collecting statistics about a second statistic of each reference sub-sample that is of the reference sample and that includes the sample fragments includes: traversing all the reference sub-samples of the reference sample, and when a reference sub-sample includes at least one basic element in the sample fragments, adding 1 to a second statistic of the reference sub-sample.

Beneficial effects of the foregoing implementation examples are: the sample fragments are properly grouped and processed by determining positions that are in the reference samples and at which sample fragments are relatively sparse, thereby improving a data processing speed and data processing efficiency while ensuring a test effect.

In another feasible implementation of the first aspect, after the combining the adjacent reference sub-samples, when a quantity of the reference sub-samples is greater than a third threshold, the method further includes: increasing the second threshold; and when a sum of second statistics of any adjacent reference sub-samples is less than the increased second threshold, combining the adjacent reference sub-samples.

Beneficial effects of this implementation are: the reference sub-samples are iteratively combined, thereby finally obtaining a degree of parallelism of data processing that meets an actual requirement.

In another feasible implementation of the first aspect, the data processing system further includes a second sample set, the second sample set includes at least two sample fragments, and before the traversing all the sample fragments in the first sample set and collecting statistics about a first statistic of each basic element that is in the reference sample and that is included in the sample fragments, the method further includes: segmenting the second sample set into at least two first sample sets.

In another feasible implementation of the first aspect, the segmenting the second sample set into at least two first sample sets includes: determining a segmentation point for the segmentation, where the segmentation point includes positions, in the reference sample, of a preset quantity of basic elements selected at equal intervals from all the basic elements in the reference sample that are sorted according to the preset order; and traversing all the sample fragments in the second sample set, and determining, according to a position of the segmentation point and a position that is in the reference sample and that is of the first one of basic elements sorted according to the preset order in the sample fragment, a first sample set to which the sample fragment belongs.

In another feasible implementation of the first aspect, the segmenting the second sample set into at least two first sample sets includes: obtaining a third sample set, where the third sample set is a subset of the second sample set, and the third sample set includes at least two sample fragments; traversing all the sample fragments in the third sample set, and determining a position that is in the reference sample and that is of the first one of basic elements sorted according to the preset order in the sample fragment; determining a segmentation point for the segmentation, where the segmentation point includes a preset quantity of positions that are selected at equal intervals from determined positions according to the preset order; and traversing all the sample fragments in the second sample set, and determining, according to a position of the segmentation point and the position that is in the reference sample and that is of the first one of the basic elements sorted according to the preset order in the sample fragment, a first sample set to which the sample fragment belongs.

In another feasible implementation of the first aspect, the division point further includes the segmentation point, and before the combining, when a sum of second statistics of any adjacent reference sub-samples is less than a second threshold, the adjacent reference sub-samples, the method further includes: combining two reference sub-samples adjacent to the segmentation point when a first statistic of a basic element on the segmentation point is greater than the first threshold.

In another feasible implementation of the first aspect, the division point further includes the segmentation point, and before the combining, when a sum of second statistics of any adjacent reference sub-samples is less than a second threshold, the adjacent reference sub-samples, the method further includes: combining two reference sub-samples adjacent to the segmentation point when a first statistic of a basic element on the segmentation point is greater than or equal to the first threshold.

Beneficial effects of the foregoing implementation examples are: the second sample set is segmented into several first sample sets, thereby improving a degree of parallelism of data processing.

In another feasible implementation of the first aspect, after the combining, when a sum of second statistics of any adjacent reference sub-samples is less than a second threshold, the adjacent reference sub-samples, the method further includes: determining a test sample set, where the test sample set includes sample fragments that are included in a same reference sub-sample; and performing subsequent data processing by using the test sample set as a basic processing unit.

In another feasible implementation of the first aspect, after the dividing the reference sample into at least two reference sub-samples, the method further includes: determining a test sample set, where the test sample set includes sample fragments that are included in a same reference sub-sample.

In another feasible implementation of the first aspect, after the combining the adjacent reference sub-samples, the method further includes: combining adjacent test sample sets, where a test sample set obtained after the combination includes a sample fragment that is included in a reference sub-sample obtained after the combination; and performing subsequent data processing by using the test sample set as a basic processing unit.

The first aspect of this embodiment of the present invention may be applied to DNA sequencing and recognition. The basic element includes nitrogenous base data of deoxyribonucleic acid, and the reference sample includes reference sequence data of deoxyribonucleic acid.

Beneficial effects of the foregoing implementation examples are: before subsequent data processing, to-be-processed data is properly divided into data processing units having a high degree of parallel processing, thereby improving a speed and efficiency of the subsequent data processing.

According to a second aspect, an embodiment of the present invention provides a data processing apparatus, applied to a data processing system. The data processing system includes a reference sample and a first sample set, the reference sample includes at least two basic elements sorted according to preset order, the first sample set includes at least one sample fragment, and the sample fragment includes at least one basic element captured from the reference sample. The apparatus includes: a first statistics collection module, configured to: traverse all the sample fragments in the first sample set, collect statistics about a first statistic of each basic element that is in the reference sample and that is included in the sample fragments, and determine that a position, in the reference sample, of a basic element whose first statistic is less than a first threshold is a spacing position; a first division module, configured to divide the reference sample into at least two reference sub-samples, where a division point for the division includes: a spacing position that is not adjacent to another spacing position, and any one of at least two adjacent spacing positions; a second statistics collection module, configured to: traverse all the sample fragments in the first sample set, and collect statistics about a second statistic of each reference sub-sample that is of the reference sample and that includes the sample fragments; and a first combination module, configured to combine, when a sum of second statistics of any adjacent reference sub-samples is less than a second threshold, the adjacent reference sub-samples.

In a feasible implementation of the second aspect, the first statistics collection module is specifically configured to: traverse all the basic elements in the reference sample, and when a basic element is included in a sample fragment, add 1 to a first statistic of the basic element.

In another feasible implementation of the second aspect, the first division module is specifically configured to: traverse all the basic elements in the reference sample, and when a first statistic of a basic element is less than the first threshold, determine that a position of the basic element in the reference sample is a spacing position; determine the division point according to the spacing position; and divide the reference sample into the at least two reference sub-samples according to the division point.

In another feasible implementation of the second aspect, the second statistics collection module is specifically configured to: traverse all the reference sub-samples of the reference sample, and when a reference sub-sample includes at least one basic element in the sample fragments, add 1 to a second statistic of the reference sub-sample.

Beneficial effects of the foregoing implementation examples are: the sample fragments are properly grouped and processed by determining positions that are in the reference samples and at which sample fragments are relatively sparse, thereby improving a data processing speed and data processing efficiency while ensuring a test effect.

In another feasible implementation of the second aspect, when a quantity of the reference sub-samples is greater than a third threshold, the first combination module is further configured to: increase the second threshold; and when a sum of second statistics of any adjacent reference sub-samples is less than the increased second threshold, combine the adjacent reference sub-samples.

Beneficial effects of this implementation are: the reference sub-samples are iteratively combined, thereby finally obtaining a degree of parallelism of data processing that meets an actual requirement.

In another feasible implementation of the second aspect, the data processing system further includes a second sample set, the second sample set includes at least two sample fragments, and the apparatus further includes a second division module, configured to segment the second sample set into at least two first sample sets.

In another feasible implementation of the second aspect, the second division module is specifically configured to: determine a segmentation point for the segmentation, where the segmentation point includes positions, in the reference sample, of a preset quantity of basic elements selected at equal intervals from all the basic elements in the reference sample that are sorted according to the preset order; and traverse all the sample fragments in the second sample set, and determine, according to a position of the segmentation point and a position that is in the reference sample and that is of the first one of basic elements sorted according to the preset order in the sample fragment, a first sample set to which the sample fragment belongs.

In another feasible implementation of the second aspect, the second division module is specifically configured to: obtain a third sample set, where the third sample set is a subset of the second sample set, and the third sample set includes at least two sample fragments; traverse all the sample fragments in the third sample set, and determine a position that is in the reference sample and that is of the first one of basic elements sorted according to the preset order in the sample fragment; determine a segmentation point for the segmentation, where the segmentation point includes a preset quantity of positions that are selected at equal intervals from determined positions according to the preset order; and traverse all the sample fragments in the second sample set, and determine, according to a position of the segmentation point and the position that is in the reference sample and that is of the first one of the basic elements sorted according to the preset order in the sample fragment, a first sample set to which the sample fragment belongs.

In another feasible implementation of the second aspect, the division point further includes the segmentation point, and the apparatus further includes a second combination module, configured to combine two reference sub-samples adjacent to the segmentation point when a first statistic of a basic element on the segmentation point is greater than the first threshold.

In another feasible implementation of the second aspect, the division point further includes the segmentation point, and the apparatus further includes a second combination module, configured to combine two reference sub-samples adjacent to the segmentation point when a first statistic of a basic element on the segmentation point is greater than or equal to the first threshold.

Beneficial effects of the foregoing implementation examples are: the second sample set is segmented into several first sample sets, thereby improving a degree of parallelism of data processing.

In another feasible implementation of the second aspect, the apparatus further includes a first determining module, configured to: determine a test sample set, where the test sample set includes sample fragments that are included in a same reference sub-sample; and perform subsequent data processing by using the test sample set as a basic processing unit.

In another feasible implementation of the second aspect, the apparatus further includes a second determining module, configured to determine a test sample set, where the test sample set includes sample fragments that are included in a same reference sub-sample.

In another feasible implementation of the second aspect, the apparatus further includes a third combination module, configured to: combine adjacent test sample sets, where a test sample set obtained after the combination includes a sample fragment that is included in a reference sub-sample obtained after the combination; and perform subsequent data processing by using the test sample set as a basic processing unit.

The second aspect of this embodiment of the present invention may be applied to DNA sequencing and recognition. The basic element includes nitrogenous base data of deoxyribonucleic acid, and the reference sample includes reference sequence data of deoxyribonucleic acid.

Beneficial effects of the foregoing implementation examples are: before subsequent data processing, to-be-processed data is properly divided into data processing units having a high degree of parallel processing, thereby improving a speed and efficiency of the subsequent data processing.

According to a third aspect, an embodiment of the present invention provides a data processing apparatus, applied to a data processing system. The data processing system includes a reference sample and a first sample set, the reference sample includes at least two basic elements sorted according to preset order, the first sample set includes at least one sample fragment, and the sample fragment includes at least one basic element captured from the reference sample. The apparatus includes a processor and a memory coupled to the processor. The memory is configured to store code. The processor is configured to perform the following operations according to the code: traversing all the sample fragments in the first sample set, collecting statistics about a first statistic of each basic element that is in the reference sample and that is included in the sample fragments, and determining that a position, in the reference sample, of a basic element whose first statistic is less than a first threshold is a spacing position; dividing the reference sample into at least two reference sub-samples, where a division point for the division includes: a spacing position that is not adjacent to another spacing position, and any one of at least two adjacent spacing positions; traversing all the sample fragments in the first sample set, and collecting statistics about a second statistic of each reference sub-sample that is of the reference sample and that includes the sample fragments; and combining, when a sum of second statistics of any adjacent reference sub-samples is less than a second threshold, the adjacent reference sub-samples

In a feasible implementation of the third aspect, the processor is specifically configured to: traverse all the basic elements in the reference sample, and when a basic element is included in a sample fragment, add 1 to a first statistic of the basic element.

In another feasible implementation of the third aspect, the processor is specifically configured to: traverse all the basic elements in the reference sample, and when a first statistic of a basic element is less than the first threshold, determine that a position of the basic element in the reference sample is a spacing position; determine the division point according to the spacing position; and divide the reference sample into the at least two reference sub-samples according to the division point.

In another feasible implementation of the third aspect, the processor is specifically configured to: traverse all the reference sub-samples of the reference sample, and when a reference sub-sample includes at least one basic element in the sample fragments, add 1 to a second statistic of the reference sub-sample.

Beneficial effects of the foregoing implementation examples are: the sample fragments are properly grouped and processed by determining positions that are in the reference samples and at which sample fragments are relatively sparse, thereby improving a data processing speed and data processing efficiency while ensuring a test effect.

In another feasible implementation of the third aspect, the processor is further configured to: increase the second threshold; and when a sum of second statistics of any adjacent reference sub-samples is less than the increased second threshold, combine the adjacent reference sub-samples.

Beneficial effects of this implementation are: the reference sub-samples are iteratively combined, thereby finally obtaining a degree of parallelism of data processing that meets an actual requirement.

In another feasible implementation of the third aspect, the data processing system further includes a second sample set, the second sample set includes at least two sample fragments, and the processor is further configured to segment the second sample set into at least two first sample sets.

In another feasible implementation of the third aspect, the processor is specifically configured to: determine a segmentation point for the segmentation, where the segmentation point includes positions, in the reference sample, of a preset quantity of basic elements selected at equal intervals from all the basic elements in the reference sample that are sorted according to the preset order; and traverse all the sample fragments in the second sample set, and determine, according to a position of the segmentation point and a position that is in the reference sample and that is of the first one of basic elements sorted according to the preset order in the sample fragment, a first sample set to which the sample fragment belongs.

In another feasible implementation of the third aspect, the processor is specifically configured to: obtain a third sample set, where the third sample set is a subset of the second sample set, and the third sample set includes at least two sample fragments; traverse all the sample fragments in the third sample set, and determine a position that is in the reference sample and that is of the first one of basic elements sorted according to the preset order in the sample fragment; determine a segmentation point for the segmentation, where the segmentation point includes a preset quantity of positions that are selected at equal intervals from determined positions according to the preset order; and traverse all the sample fragments in the second sample set, and determine, according to a position of the segmentation point and the position that is in the reference sample and that is of the first one of the basic elements sorted according to the preset order in the sample fragment, a first sample set to which the sample fragment belongs.

In another feasible implementation of the third aspect, the division point further includes the segmentation point, and the processor is further configured to combine two reference sub-samples adjacent to the segmentation point when a first statistic of a basic element on the segmentation point is greater than the first threshold.

In another feasible implementation of the third aspect, the division point further includes the segmentation point, and the processor is further configured to combine two reference sub-samples adjacent to the segmentation point when a first statistic of a basic element on the segmentation point is greater than or equal to the first threshold.

Beneficial effects of the foregoing implementation examples are: the second sample set is segmented into several first sample sets, thereby improving a degree of parallelism of data processing.

In another feasible implementation of the third aspect, the processor is further configured to: determine a test sample set, where the test sample set includes sample fragments that are included in a same reference sub-sample; and perform subsequent data processing by using the test sample set as a basic processing unit.

In another feasible implementation of the third aspect, the processor is further configured to determine a test sample set, where the test sample set includes sample fragments that are included in a same reference sub-sample.

In another feasible implementation of the third aspect, the processor is further configured to: combine adjacent test sample sets, where a test sample set obtained after the combination includes a sample fragment that is included in a reference sub-sample obtained after the combination; and perform subsequent data processing by using the test sample set as a basic processing unit.

The third aspect of this embodiment of the present invention may be applied to DNA sequencing and recognition. The basic element includes nitrogenous base data of deoxyribonucleic acid, and the reference sample includes reference sequence data of deoxyribonucleic acid.

Beneficial effects of the foregoing implementation examples are: before subsequent data processing, to-be-processed data is properly divided into data processing units having a high degree of parallel processing, thereby improving a speed and efficiency of the subsequent data processing.

According to a fourth aspect, an embodiment of the present invention provides a computer readable storage medium storing an instruction, where when the instruction is executed, one or more processors of a device that performs data task allocation on to-be-processed data are used to perform the method according to the first aspect and the feasible implementations of the first aspect.

Beneficial effects of this implementation are: the sample fragments are properly grouped and processed by determining positions that are in the reference samples and at which sample fragments are relatively sparse, thereby improving a data processing speed and data processing efficiency while ensuring a test effect.

It should be understood that the embodiments of the present invention may be further used for task scheduling, allocation, distribution, and the like in the data processing field. For example, the reference sample may alternatively be a to-be-recognized object having a set of features, for example, an image or a sound signal. The corresponding basic element includes each feature amount. The features may be classified and sorted according to resource consumption of each feature amount in data processing. Further, a degree of parallelism of data processing is improved by using the method disclosed in the embodiments of the present invention, thereby improving a speed and efficiency of a data processing process such as feature matching, model training, and object recognition.

### BRIEF DESCRIPTION OF DRAWINGS

To describe the technical solutions in the embodiments of the present invention or in the prior art more clearly, the following briefly describes the accompanying drawings required for describing the embodiments or the prior art. Apparently, the accompanying drawings in the following description show merely some embodiments of the present invention, and a person of ordinary skill in the art may still derive other drawings from these accompanying drawings without creative efforts.
FIG. 1 is a schematic diagram of an implementation of data balancing according to an embodiment of the present invention;
FIG. 2 is a schematic diagram of a distributed end-to-end gene detection system according to an embodiment of the present invention;
FIG. 3 is a schematic diagram of a data processing method according to an embodiment of the present invention;
FIG. 4 is a schematic diagram of combining reference sub-samples according to an embodiment of the present invention;
FIG. 5 is a block diagram of an example of a data processing apparatus according to an embodiment of the present invention; and
FIG. 6 is a block diagram of an example of another data processing apparatus according to an embodiment of the present invention.

### DESCRIPTION OF EMBODIMENTS

The following clearly and completely describes the technical solutions in the embodiments of the present invention with reference to the accompanying drawings in the embodiments of the present invention. Apparently, the described embodiments are merely some but not all of the embodiments of the present invention.

To facilitate clear description of the technical solutions in the embodiments of the present invention, words such as "first", "second", and "third" are used in the embodiments of the present invention to distinguish between same items or similar items that provide basically same functions and purposes. A person skilled in the art may understand that the words such as "first", "second", and "third" do not limit a quantity and execution order.

A high throughput sequencer usually outputs reads having a length of tens to hundreds of nitrogenous bases, and a human genome includes approximately 3 billion of nitrogenous bases. Assuming that an average read length is 100 nitrogenous bases, at a 60x sequencing depth, nitrogenous base characters of approximately 180 GB can be output, which correspond to 1.8 billion of reads. In a genome analysis pipeline, each read needs to be compared with a reference sequence having a length of approximately 3 billion of characters, and mapped to chromosome coordinates having a highest matching probability. Because the sequencer has a sequencing preference for DNA, and DNA has repeated nitrogenous base sequences, sequencing reads obtained after the mapping are usually in skewed distribution, and consequently data skew and computation skew are caused to a variant calling process in different chromosome regions. The data skew mainly refers to imbalance between total quantities of reads in chromosome regions, and is externally manifested as a difference between data volumes of read files. The computation skew mainly refers to imbalance between time overheads required for analyzing reads per unit quantity in different chromosome regions.

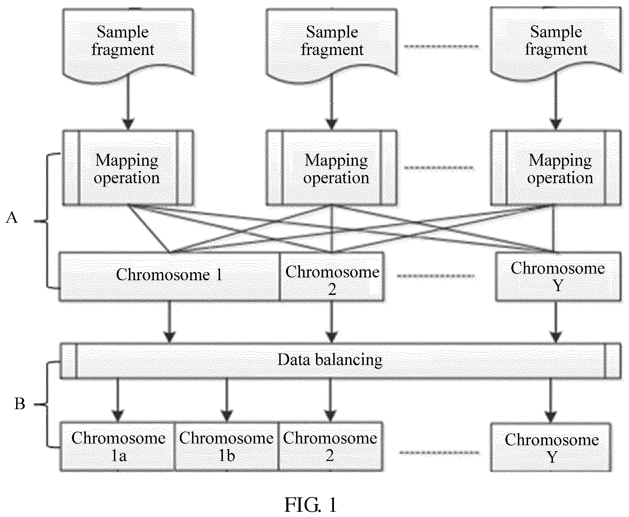

In a feasible implementation, a data balancing module is added after a mapping operation is performed on DNA sequencing data. A result record of the mapping operation is first classified, according to a chromosome on which the result record is located, as a data group (as shown by part A in FIG. 1) represented by the corresponding chromosome. Then, statistics about a quantity of records in a data group represented by each chromosome are collected and a statistical result is transferred to the data balancing module. Finally, the data balancing module divides, according to the quantity of records in each data group, a data group in which data skew occurs into two data subgroups (as shown by part B in FIG. 1, where a data group represented by a chromosome 1 has a large data volume, that is, data skew occurs, and therefore is divided into two data subgroups: a chromosome 1a and a chromosome 1b). Each data subgroup that is processed by the data balancing module is correspondingly one subtask, and the subtasks are started and executed in parallel in a computing cluster. Processing data in parallel improves a data processing speed and data processing efficiency. However, simply dividing a data group in which skew occurs still has data division blindness, thereby limiting a degree of task parallelism. In addition, in the division manner in the solution, a boundary of a chromosome region may be at a chromosome position with high coverage, thereby exerting negative impact on accuracy of a variant detection result.

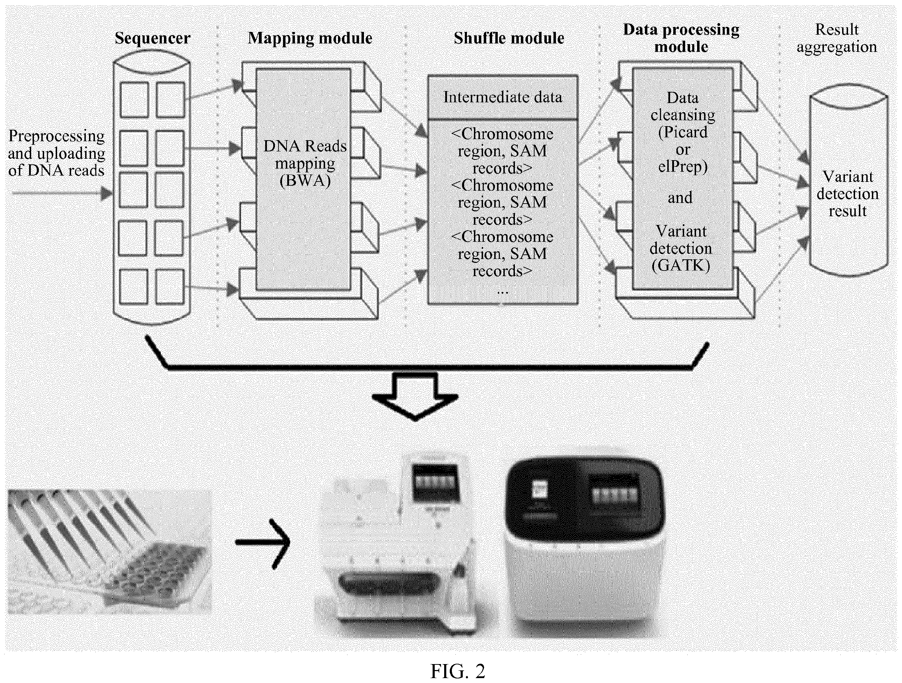

FIG. 2 is a schematic diagram of a distributed end-to-end genome detection system according to an embodiment of the present invention. (1) Read data output by a sequencer is divided into multiple pieces and stored in a distributed file system. (2) A mapping module maps a read in the distributed file system to a reference gene sequence, and outputs a mapping result. (3) A shuffle (Shuffle) module distributes the read data to a corresponding data processing (Reduce) module according to the mapping result, and each data processing module corresponds to a preset genome processing region. (4) The data processing module performs steps of data cleansing and variant calling on the received data, and the data cleansing includes intermediate steps such as deduplication, local rearrangement, and nitrogenous base quality correction. (5) Variant calling results of the data processing modules are merged and output. In an actual product, the foregoing modules may exist in a same gene sequencer or a gene diagnostic instrument. After a to-be-sequenced gene sample is input, the gene sequencer or the gene diagnostic instrument automatically completes the foregoing data processing steps, and outputs a detection result. It should be understood that according to the division of the foregoing functional modules, a basic principle of the distributed genome detection system is described by way of example. In an actual product, different specific hardware or software implementations may be used to implement a same system function, and are not limited. In this embodiment of the present invention, a data division solution is introduced into the shuffle module of the system, so that data is more properly distributed, and a data processing speed and data processing efficiency of the data processing module is improved while accuracy of a data processing result is ensured.

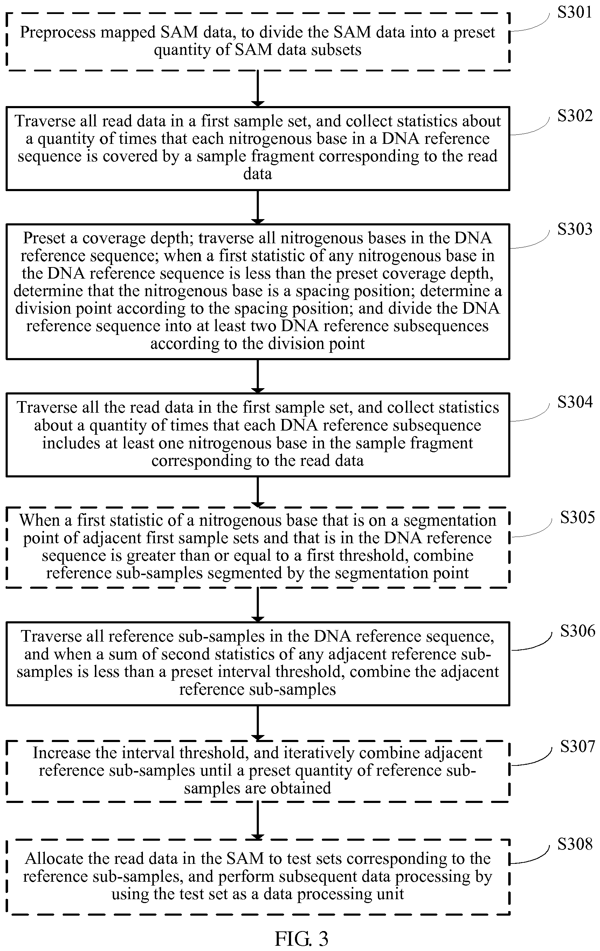

As shown in FIG. 3, an embodiment of the present invention provides a data processing method.

S301: Preprocess mapped SAM data, to divide the SAM data into a preset quantity of SAM data subsets.

In this embodiment of the present invention, all mapped SAM data is referred to as a second sample set, and the SAM data subset obtained after the division is referred to as a first sample set. A reference sample is a selected DNA reference sequence. Each piece of SAM data, that is, each piece of read data, is a sample fragment of the selected DNA reference sequence. A nitrogenous base is a basic element for constituting a DNA sequence or a DNA sequence fragment, and nitrogenous bases in a DNA reference sequence are sequentially sorted according to specified order in the DNA. Coordinates, in a selected DNA reference sequence, of the first nitrogenous base in a sample fragment represented by read data are referred to as coordinates of the read data or coordinates of the sample fragment represented by the read data. In a feasible implementation, first, all read data in the second sample set may be sorted according to coordinates of the read data. Then, all sorted SAM data in the second sample set is divided into the preset quantity of SAM subsets. Assuming that the preset quantity is N, the SAM data subsets may be sequentially referred to as a first sample set 1, a first sample set 2, a first sample set 3, ..., and a first sample set N. It can be understood that read data in each first sample set is sorted according to coordinates of the read data, and the first sample sets are also sorted according to the foregoing order.

In a feasible implementation, the second sample set is segmented into at least two first sample sets, including: determining a segmentation point for the segmentation, where the segmentation point includes positions, in the reference sample, of a preset quantity of basic elements selected at equal intervals from all basic elements in the reference sample that are sorted according to preset order; and traversing all sample fragments in the second sample set, and determining, according to a position of the segmentation point and a position that is in the reference sample and that is of the first one of basic elements sorted according to the preset order in the sample fragment, a first sample set to which the sample fragment belongs.

Specifically, S301 includes the following steps.

S30111: Collect statistics about a length of a selected DNA reference sequence, that is, a total quantity L of included nitrogenous bases, where L is a positive integer.

S30112: If it is preset that a second sample set is divided into R first sample sets, determine that a quantity of samples in each first sample set is └L/R┘ or ┌L/R┐, where └.┘ and ┌.┐ respectively indicate rounding down and rounding up.

S30113: Assume M=L%R, where % indicates a modulo operation, determine a division every └L/R┘ from a position of the first pair of nitrogenous bases in the DNA reference sequence, until an (R-M)^{th} division is determined, and determine a division every [L/R] from an (R-M+1)^{th} division, until an R^{th} division is determined.

S30114: Distribute all read data in a SAM file to corresponding divisions according to coordinates of the read data, where a SAM data subset included in each division is a first data set. Read data in the SAM data subset may be sorted during the distribution, or read data in the SAM data subset may be sorted after the SAM data subset is obtained. A sorting basis is the same as that in the foregoing description, and details are not described again.

In another feasible implementation, the second sample set is segmented into at least two first sample sets, including: obtaining a third sample set, where the third sample set is a subset of the second sample set, and the third sample set includes at least two sample fragments; traversing all the sample fragments in the third sample set, and determining a position that is in the reference sample and that is of the first one of basic elements sorted according to preset order in the sample fragment; determining a segmentation point for the segmentation, where the segmentation point includes a preset quantity of positions that are selected at equal intervals from determined positions according to the preset order; and traversing all sample fragments in the second sample set, and determining, according to a position of the segmentation point and the position that is in the reference sample and that is of the first one of the basic elements sorted according to the preset order in the sample fragment, a first sample set to which the sample fragment belongs.

Specifically, it can be understood that the third sample set is a subset of the second sample set, and S301 includes the following steps.

S30121: Randomly extract a preset amount of read data from all read data in a second sample set, to form a third sample set. It is assumed that the preset amount is S, and the read data in the third sample set is sorted according to coordinates. A sorting process may be performed during formation of the third sample set, or may be performed after formation of the third sample set. That is, the read data in the third sample set is sorted. A sorting basis is the same as that in the foregoing description, and details are not described again.

S30122: If it is preset that the second sample set is divided into R first sample sets, determine that a quantity of samples in each division is └S/R┘ or ┌S/R┐, where └.┘ and ┌.┐ respectively indicate rounding down and rounding up.

S30123: Assume M=S%R, where % indicates a modulo operation, determine a division every └S/R┘ from a position of a first pair of nitrogenous bases in a DNA reference sequence, until an (R-M)^{th} division is determined, and determine a division every ┌S/R┐ from an (R-M+1)^{th} division, until an R^{th} division is determined.

S30124: Distribute all read data in a SAM file to corresponding divisions according to coordinates of the read data, where a SAM data subset included in each division is a first data set. Read data in the SAM data subset may be sorted during the distribution, or read data in the SAM data subset may be sorted after the SAM data subset is obtained. A sorting basis is the same as that in the foregoing description, and details are not described again.

Step S301 of segmenting the second sample set into several first sample sets improves a degree of parallelism of data processing. It should be understood that the foregoing segmentation operation may alternatively not be performed, and the second sample set is considered as a first sample set for subsequent processing. That is, in a feasible implementation of this embodiment of the present invention, step S301 is an optional step.

S302: Traverse all read data in a first sample set, and collect statistics about a quantity of times that each nitrogenous base in a DNA reference sequence is covered by a sample fragment corresponding to the read data.

It should be understood that when step S301 is not performed, the first sample set in this step is the second sample set, that is, read data in all the SAM data. When step S301 is performed, the first sample set in this step is a subset of all the SAM data. In this step, operations of traversing all read data in the first sample set and collecting statistics about the quantity of times that each nitrogenous base in the DNA reference sequence is covered by the sample fragment corresponding to the read data need to be performed for all the R first sample sets.

The quantity of times that each nitrogenous base in the DNA reference sequence is covered by the sample fragment corresponding to the read data is referred to as a first statistic. The statistics collection operation includes: traversing all the basic elements in the reference sample, and when a basic element is included in the sample fragment, adding 1 to a first statistic of the basic element. Specifically, statistics about a first statistic are collected for each nitrogenous base in the DNA reference sequence. It is assumed that statistics about a first statistic n are collected for an n^{th} nitrogenous base, where n is a positive integer. When a sample fragment corresponding to any read data includes an n^{th} nitrogenous base, 1 is added to the first statistic n, and the first statistic is obtained until all the read data is traversed.

S303: Preset a coverage depth; traverse all nitrogenous bases in the DNA reference sequence; when a first statistic of any nitrogenous base in the DNA reference sequence is less than the preset coverage depth, determine that the nitrogenous base is a spacing position; determine a division point according to the spacing position; and divide the DNA reference sequence into at least two DNA reference subsequences according to the division point.

The preset coverage depth is referred to as a first threshold. The first threshold may be preset according to an actual requirement. The DNA reference subsequence is referred to as a reference sub-sample. All the nitrogenous bases in the DNA reference sequence are traversed. When the first statistic, obtained in step S302, of any nitrogenous base is less than the first threshold, it is determined that a position of the nitrogenous base is the spacing position. When any spacing position is not adjacent to another spacing position, the spacing position is a division point. When any spacing position is adjacent to at least one another spacing position, any one of the spacing positions is selected as a division point. For example, when a spacing position A, a spacing position B, and a spacing position C are sorted in sequence and adjacent, any position of A, B, or C may be selected as a division point. This is not limited. All the nitrogenous bases in the DNA reference sequence are traversed from the first one of the nitrogenous bases sorted in the DNA reference sequence. Each time a division point is found, a reference sub-sample is determined. The last reference sub-sample is determined when the last nitrogenous base in the DNA reference sequence is found. Each nitrogenous base on a division point may belong to any one of adjacent reference sub-samples, and this is not limited. For example, each nitrogenous base on a division point belongs to adjacent reference sub-samples that are sorted in front.

A nitrogenous base between the first one of the nitrogenous bases sorted in the DNA reference sequence and the first division point forms the first reference sub-sample. A nitrogenous base between the last one of the nitrogenous bases sorted in the DNA reference sequence and the last division point forms the last reference sub-sample. A nitrogenous base between other adjacent division points forms another reference sub-sample.

It should be understood that in another feasible implementation, according to an actual requirement, step S303 may be: presetting a coverage depth; traversing all nitrogenous bases in the DNA reference sequence; when a first statistic of any nitrogenous base in the DNA reference sequence is less than or equal to the preset coverage depth, determining that the nitrogenous base is a spacing position, where a spacing position that is not adjacent to another spacing position and any one of at least two adjacent spacing positions are division points; and dividing the DNA reference sequence into at least two DNA reference subsequences according to the division points.

S304: Traverse all the read data in the first sample set, and collect statistics about a quantity of times that each DNA reference subsequence includes at least one nitrogenous base in the sample fragment corresponding to the read data.

It should be understood that when step S301 is not performed, the first sample set in this step is the second sample set, that is, read data in all the SAM data. When step S301 is performed, the first sample set in this step is a subset of all the SAM data. In this step, operations of traversing all the read data in the first sample set and collecting statistics about the quantity of times that each DNA reference subsequence includes at least one nitrogenous base in the sample fragment corresponding to the read data need to be performed for all the R first sample sets.

The quantity of times that each DNA reference subsequence includes at least one nitrogenous base in the sample fragment corresponding to the read data is referred to as a second statistic. The statistics collection operation includes: traversing all reference sub-samples of the reference sample, and when a reference sub-sample includes at least one basic element in the sample fragments, adding 1 to a second statistic of the reference sub-sample. Specifically, statistics about a second statistic are collected for each DNA reference subsequence, that is, each reference sub-sample. It is assumed that statistics about a second statistic m are collected for an m^{th} reference sub-sample, where m is a positive integer. When the m^{th} reference sub-sample includes any nitrogenous base the same as that in a sample fragment corresponding to any piece of read data, 1 is added to the second statistic m, and the second statistic is obtained after all the read data is traversed.

S305: When a first statistic of a nitrogenous base that is on a segmentation point of adjacent first sample sets and that is in the DNA reference sequence is greater than a first threshold, combine reference sub-samples segmented by the segmentation point.

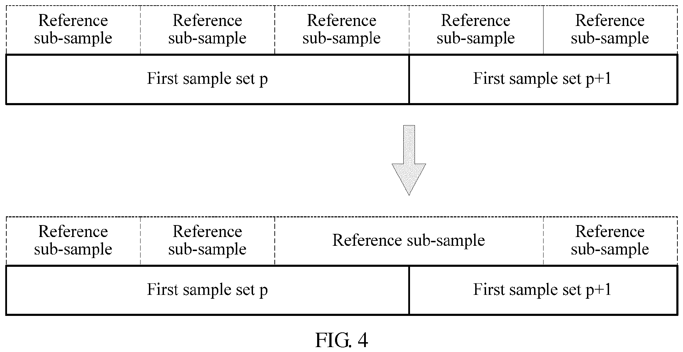

It should be understood that step 301 is a premise for performing this step. When step 301 is not performed, this step is not performed either. When step 301 is performed, step 303 of obtaining reference sub-samples by means of division is performed in each first sample set. Therefore, for the DNA reference sequence, a segmentation point of adjacent first sample sets is also a division point of the reference sub-samples. When step 302 is performed, a first statistic of each nitrogenous base in the DNA reference sequence is obtained, so that a first statistic of a nitrogenous base that is on a segmentation point of adjacent first sample sets and that is in the DNA reference sequence is obtained. When the first statistic is greater than the first threshold, two reference sub-samples adjacent to the segmentation point are combined into one reference sub-sample, and second statistics, obtained in step 304, of the two combined reference sub-samples that are adjacent are added. A sum of the second statistics is used as a second statistic of a reference sub-sample obtained after the combination. FIG. 4 shows a process of combining the last reference sub-sample in a p^{th} first sample set and the first reference sub-sample in a (p+1)^{th} first sample set.

It should be understood that in another feasible implementation, according to an actual requirement, step S305 may be: when a first statistic of a nitrogenous base that is on a segmentation point of adjacent first sample sets and that is in the DNA reference sequence is greater than or equal to a first threshold, combining reference sub-samples segmented by the segmentation point.

Generally, comparing and determining the first statistic and the first threshold during the combination of the reference sub-samples in step S305 is consistent with comparing and determining the first statistic and the first threshold during the obtaining of the reference sub-samples by means of division. For example, in S301, when the first statistic is less than the first threshold, the reference sub-samples are obtained by means of division; in S05, when the first statistic is greater than or equal to the first threshold, the reference sub-samples are combined, and vice verse.

S306: Traverse all reference sub-samples in the DNA reference sequence, and when a sum of second statistics of any adjacent reference sub-samples is less than a preset interval threshold, combine the adjacent reference sub-samples.

It is assumed that the preset interval threshold is a second threshold. The second threshold may be preset according to an actual requirement. Specifically, all the reference sub-samples in the DNA reference sequence are traversed. When a sum of second statistics of adjacent reference sub-samples is less than the second threshold, the adjacent reference sub-samples are combined into one reference sub-sample, and the sum of the second statistics is used as a second statistic of the reference sub-sample obtained after the combination, until all the reference subsequences are traversed.

S307: Increase the interval threshold, and iteratively combine adjacent reference sub-samples until a preset quantity of reference sub-samples are obtained.

It should be understood that step 307 is an optional step, and may be performed or not performed according to an actual requirement. It is assumed that the preset quantity is a third threshold, and the third threshold may be preset according to an actual requirement.

Specifically, the second threshold may be increased by a specific step, and step 306 is performed according to the increased second threshold, to obtain a new reference sub-sample. Steps are iteratively performed, until a total quantity of obtained reference sub-samples is the same as the third threshold or is within an allowable difference range.

S308: Allocate the read data in the SAM to test sets corresponding to the reference sub-samples, and perform subsequent data processing by using the test set as a data processing unit.

According to step S301 to step S307, a data processing unit in a form of a reference sub-sample is determined, and data processing is performed according to the data processing unit. This improves a data processing speed and data processing efficiency while ensuring test quality.

In a feasible implementation, the data processing unit is determined after the reference sub-sample is determined, including: determining a test sample set, where the test sample set includes sample fragments that are included in a same reference sub-sample; and performing subsequent data processing by using the test sample set as a basic processing unit. Specifically, all the read data in the SAM is categorized, according to coordinates of the read data, into test sample sets corresponding to different reference sub-samples. Read data corresponding to a same reference sub-sample belongs to a same test sample set. Subsequent data processing steps are performed by using the test sample set as a data processing unit.

In another feasible implementation, the data processing unit is determined at the same time as the reference sub-sample is determined, including: after step S303, determining a test sample set, where the test sample set includes sample fragments that are included in a same reference sub-sample; and after step S307, combining adjacent test sample sets, where a test sample set obtained after the combination includes a sample fragment that is included in a reference sub-sample obtained after the combination, and performing subsequent data processing by using the test sample set as a basic processing unit. Specifically, after the dividing the DNA reference sequence into at least two DNA reference subsequences in step S303, all the read data in the SAM is categorized, according to coordinates of the read data, into test sample sets corresponding to different reference sub-samples. Read data corresponding to a same reference sub-sample belongs to a same test sample set. Then, in steps S305, S306, and S307, each time adjacent reference sub-samples are combined, test sample sets corresponding to the adjacent reference sub-samples are combined, and subsequent data processing steps are performed by using a test sample set as a data processing unit.

In this embodiment of the present invention, DNA mapping data is properly grouped and processed by determining positions that are in the DNA reference sequence and at which DNA mapping data is relatively sparse. This improves a data processing speed and data processing efficiency while ensuring a test effect.

It should be understood that this embodiment of the present invention may be further used for task scheduling, allocation, distribution, and the like in the data processing field. For example, the reference sample may alternatively be a to-be-recognized object having a set of features, for example, an image or a sound signal. The corresponding basic element includes each feature amount. The features may be classified and sorted according to resource consumption of each feature amount in data processing. Further, a degree of parallelism of data processing is improved by using the method disclosed in this embodiment of the present invention, thereby improving a speed and efficiency of a data processing process such as feature matching, model training, and object recognition.

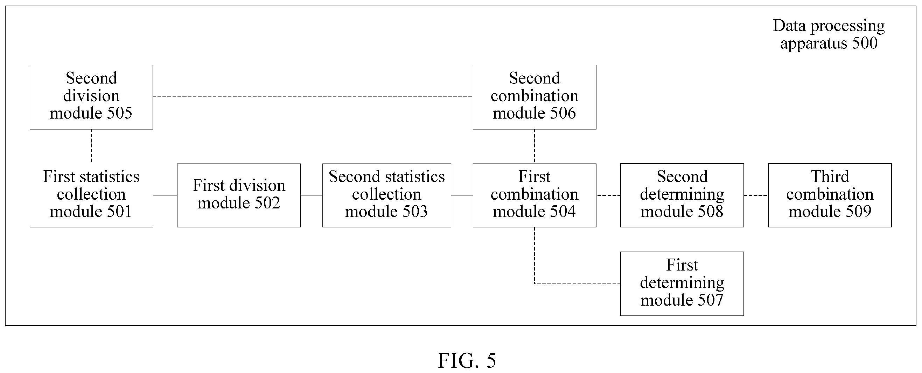

As shown in FIG. 5, an embodiment of the present invention provides a data processing apparatus 500, applied to a data processing system. The data processing system includes a reference sample and a first sample set, the reference sample includes at least two basic elements sorted according to preset order, the first sample set includes at least one sample fragment, and the sample fragment includes at least one basic element captured from the reference sample. The apparatus includes a first statistics collection module 501, a first division module 502, a second statistics collection module 503, and a first combination module 504.

The first statistics collection module 501 is configured to: traverse all the sample fragments in the first sample set, collect statistics about a first statistic of each basic element that is in the reference sample and that is included in the sample fragments, and determine that a position, in the reference sample, of a basic element whose first statistic is less than a first threshold is a spacing position; and is specifically configured to: traverse all the basic elements in the reference sample, and when a basic element is included in a sample fragment, add 1 to a first statistic of the basic element. For example, the first statistics collection module may perform step S302. For a specific implementation, refer to S302, and details are not described again.

The first division module 502 is configured to divide the reference sample into at least two reference sub-samples, where a division point for the division includes: a spacing position that is not adjacent to another spacing position, and any one of at least two adjacent spacing positions; and is specifically configured to: traverse all the basic elements in the reference sample, and when a first statistic of a basic element is less than the first threshold, determine that a position of the basic element in the reference sample is a spacing position; determine the division point according to the spacing position; and divide the reference sample into the at least two reference sub-samples according to the division point. For example, the first division module may perform step S303. For a specific implementation, refer to S303, and details are not described again.

The second statistics collection module 503 is configured to: traverse all the sample fragments in the first sample set, and collect statistics about a second statistic of each reference sub-sample that is of the reference sample and that includes the sample fragments; and is specifically configured to: traverse all the reference sub-samples of the reference sample, and when a reference sub-sample includes at least one basic element in the sample fragments, add 1 to a second statistic of the reference sub-sample. For example, the second statistics collection module may perform step S304. For a specific implementation, refer to S304, and details are not described again.

The first combination module 504 is configured to combine, when a sum of second statistics of any adjacent reference sub-samples is less than a second threshold, the adjacent reference sub-samples. For example, the first combination module may perform step S306. For a specific implementation, refer to S306, and details are not described again.

In a feasible implementation, the first combination module 504 is further configured to: increase the second threshold; and when a sum of second statistics of any adjacent reference sub-samples is less than the increased second threshold, combine the adjacent reference sub-samples. For example, the first combination module may perform step S307. For a specific implementation, refer to S307, and details are not described again.

In a feasible implementation, the data processing system further includes a second sample set, the second sample set includes at least two sample fragments, and the apparatus further includes a second division module 505, configured to segment the second sample set into at least two first sample sets. For example, the second division module may perform step S301. For a specific implementation, refer to S301, and details are not described again.

In a feasible implementation, the second division module 505 is specifically configured to: determine a segmentation point for the segmentation, where the segmentation point includes positions, in the reference sample, of a preset quantity of basic elements selected at equal intervals from all the basic elements in the reference sample that are sorted according to the preset order; and traverse all the sample fragments in the second sample set, and determine, according to a position of the segmentation point and a position that is in the reference sample and that is of the first one of basic elements sorted according to the preset order in the sample fragment, a first sample set to which the sample fragment belongs. For example, the second division module may perform steps S30111 to S30114. For a specific implementation, refer to S30111 to S30114, and details are not described again.

In another feasible implementation, the second division module 505 is specifically configured to: obtain a third sample set, where the third sample set is a subset of the second sample set, and the third sample set includes at least two sample fragments; traverse all the sample fragments in the third sample set, and determine a position that is in the reference sample and that is of the first one of basic elements sorted according to the preset order in the sample fragment; determine a segmentation point for the segmentation, where the segmentation point includes a preset quantity of positions that are selected at equal intervals from determined positions according to the preset order; and traverse all the sample fragments in the second sample set, and determine, according to a position of the segmentation point and the position that is in the reference sample and that is of the first one of the basic elements sorted according to the preset order in the sample fragment, a first sample set to which the sample fragment belongs. For example, the second division module may perform steps S30121 to S30124. For a specific implementation, refer to S30121 to S30124, and details are not described again.

In a feasible implementation, the division point further includes the segmentation point, and the apparatus further includes a second combination module 506, configured to combine two reference sub-samples adjacent to the segmentation point when a first statistic of a basic element on the segmentation point is greater than the first threshold. For example, the second combination module may perform step S305. For a specific implementation, refer to S305, and details are not described again.

In a feasible implementation, the apparatus further includes a first determining module 507, configured to: determine a test sample set, where the test sample set includes sample fragments that are included in a same reference sub-sample; and perform subsequent data processing by using the test sample set as a basic processing unit. For example, the first determining module may perform a feasible implementation of step S308. For a specific implementation, refer to the feasible implementation of S308, and details are not described again.

In a feasible implementation, the apparatus further includes a second determining module 508 and a third combination module 509. The second determining module is configured to determine a test sample set, where the test sample set includes sample fragments that are included in a same reference sub-sample. The third combination module is configured to: combine adjacent test sample sets, where a test sample set obtained after the combination includes a sample fragment that is included in a reference sub-sample obtained after the combination; and perform subsequent data processing by using the test sample set as a basic processing unit. For example, the second determining module and the third combination module may perform another feasible implementation of step S308. For a specific implementation, refer to the another feasible implementation of S308, and details are not described again.

In this embodiment of the present invention, DNA mapping data is properly grouped and processed by determining positions that are in the DNA reference sequence and at which DNA mapping data is relatively sparse. This improves a data processing speed and data processing efficiency while ensuring a test effect.

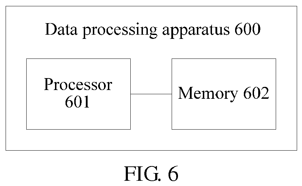

As shown in FIG. 6, an embodiment of the present invention provides a data processing apparatus 600, applied to a data processing system. The data processing system includes a reference sample and a first sample set, the reference sample includes at least two basic elements sorted according to preset order, the first sample set includes at least one sample fragment, and the sample fragment includes at least one basic element captured from the reference sample. The apparatus includes a processor 601 and a memory 602 coupled to the processor. The memory is configured to store code. The processor is configured to perform the following operations according to the code: traversing all the sample fragments in the first sample set, collecting statistics about a first statistic of each basic element that is in the reference sample and that is included in the sample fragments, and determining that a position, in the reference sample, of a basic element whose first statistic is less than a first threshold is a spacing position; dividing the reference sample into at least two reference sub-samples, where a division point for the division includes: a spacing position that is not adjacent to another spacing position, and any one of at least two adjacent spacing positions; traversing all the sample fragments in the first sample set, and collecting statistics about a second statistic of each reference sub-sample that is of the reference sample and that includes the sample fragments; and combining, when a sum of second statistics of any adjacent reference sub-samples is less than a second threshold, the adjacent reference sub-samples.

In a feasible implementation, the processor is specifically configured to: traverse all the basic elements in the reference sample, and when a basic element is included in a sample fragment, add 1 to a first statistic of the basic element.

In a feasible implementation, the processor is specifically configured to: traverse all the basic elements in the reference sample, and when a first statistic of a basic element is less than the first threshold, determine that a position of the basic element in the reference sample is a spacing position; determine the division point according to the spacing position; and divide the reference sample into the at least two reference sub-samples according to the division point.

In a feasible implementation, the processor is specifically configured to: traverse all the reference sub-samples of the reference sample, and when a reference sub-sample includes at least one basic element in the sample fragments, add 1 to a second statistic of the reference sub-sample.

In a feasible implementation, when a quantity of the reference sub-samples is greater than a third threshold, the processor is further configured to: increase the second threshold; and when a sum of second statistics of any adjacent reference sub-samples is less than the increased second threshold, combine the adjacent reference sub-samples.

In a feasible implementation, the data processing system further includes a second sample set, the second sample set includes at least two sample fragments, and the processor is further configured to segment the second sample set into at least two first sample sets.

In a feasible implementation, the processor is specifically configured to: determine a segmentation point for the segmentation, where the segmentation point includes positions, in the reference sample, of a preset quantity of basic elements selected at equal intervals from all the basic elements in the reference sample that are sorted according to the preset order; and traverse all the sample fragments in the second sample set, and determine, according to a position of the segmentation point and a position that is in the reference sample and that is of the first one of basic elements sorted according to the preset order in the sample fragment, a first sample set to which the sample fragment belongs.

In a feasible implementation, the processor is specifically configured to: obtain a third sample set, where the third sample set is a subset of the second sample set, and the third sample set includes at least two sample fragments; traverse all the sample fragments in the third sample set, and determine a position that is in the reference sample and that is of the first one of basic elements sorted according to the preset order in the sample fragment; determine a segmentation point for the segmentation, where the segmentation point includes a preset quantity of positions that are selected at equal intervals from determined positions according to the preset order; and traverse all the sample fragments in the second sample set, and determine, according to a position of the segmentation point and the position that is in the reference sample and that is of the first one of the basic elements sorted according to the preset order in the sample fragment, a first sample set to which the sample fragment belongs.

In a feasible implementation, the division point further includes the segmentation point, and the processor is specifically configured to combine two reference sub-samples adjacent to the segmentation point when a first statistic of a basic element on the segmentation point is greater than the first threshold.

In a feasible implementation, the processor is further configured to: determine a test sample set, where the test sample set includes sample fragments that are included in a same reference sub-sample; and perform subsequent data processing by using the test sample set as a basic processing unit.

In a feasible implementation, the processor is further configured to determine a test sample set, where the test sample set includes sample fragments that are included in a same reference sub-sample.

In a feasible implementation, the processor is further configured to: combine adjacent test sample sets, where a test sample set obtained after the combination includes a sample fragment that is included in a reference sub-sample obtained after the combination; and perform subsequent data processing by using the test sample set as a basic processing unit.

For example, the processor may perform the method described in step 301 to step 308. For a specific implementation, refer to step 301 to step 308, and details are not described again.

In this embodiment of the present invention, DNA mapping data is properly grouped and processed by determining positions that are in the DNA reference sequence and at which DNA mapping data is relatively sparse. This improves a data processing speed and data processing efficiency while ensuring a test effect.

It may be clearly understood by a person skilled in the art that, for the purpose of convenient and brief description, division of the foregoing function modules is taken as an example for illustration. In actual application, the foregoing functions can be allocated to different function modules and implemented according to a requirement, that is, an inner structure of an apparatus is divided into different function modules to implement all or some of the functions described above. For a detailed working process of the foregoing system, apparatus, and unit, refer to a corresponding process in the foregoing method embodiments, and details are not described herein again.

In the several embodiments provided in this application, it should be understood that the disclosed system, apparatus, and method may be implemented in other manners. For example, the described apparatus embodiment is merely an example. For example, the module or unit division is merely logical function division and may be other division in actual implementation. For example, multiple units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic or other forms.

The units described as separate parts may or may not be physically separate, and parts displayed as units may or may not be physical units, may be located in one position, or may be distributed on multiple network units. Some or all of the units may be selected according to actual requirements to achieve the objectives of the solutions of the embodiments.

In addition, functional units in the embodiments of the present invention may be integrated into one processing unit, or each of the units may exist alone physically, or two or more units are integrated into one unit. The integrated unit may be implemented in a form of a software functional unit.

When the integrated unit is implemented in the form of a software functional unit and sold or used as an independent product, the integrated unit may be stored in a computer-readable storage medium. Based on such an understanding, all or some of the technical solutions may be implemented in a form of a software product. The software product is stored in a storage medium, and includes several instructions for instructing a computer device (which may be a personal computer, a server, or a network device) to perform all or some of the steps of the methods described in the embodiments of the present invention. The storage medium is a non-transitory (non-transitory) medium, including various media that can store a program, such as a flash memory, a removable hard disk, a read-only memory, a random access memory, a magnetic disk, or an optical disc.

The foregoing descriptions are merely specific implementations of the present invention, but are not intended to limit the protection scope of the present invention. Any variation or replacement readily figured out by a person skilled in the art within the technical scope disclosed in the present invention shall fall within the protection scope of the present invention. Therefore, the protection scope of the present invention shall be subject to the protection scope of the claims.

## Claims

1. A data processing method, applied to a data processing system, wherein the data processing system comprises a reference sample and a first sample set, the reference sample comprises at least two basic elements sorted according to preset order, the first sample set comprises at least one sample fragment, and the sample fragment comprises at least one basic element captured from the reference sample; and the method comprises:
traversing all the sample fragments in the first sample set, collecting statistics about a first statistic of each basic element that is in the reference sample and that is comprised in the sample fragments, and determining that a position, in the reference sample, of a basic element whose first statistic is less than a first threshold is a spacing position;
dividing the reference sample into at least two reference sub-samples, wherein a division point for the division comprises: a spacing position that is not adjacent to another spacing position, and any one of at least two adjacent spacing positions;
traversing all the sample fragments in the first sample set, and collecting statistics about a second statistic of each reference sub-sample that is of the reference sample and that comprises the sample fragments; and
combining, when a sum of second statistics of any adjacent reference sub-samples is less than a second threshold, the adjacent reference sub-samples.

2. The method according to claim 1, wherein the collecting statistics about a first statistic of each basic element that is in the reference sample and that is comprised in the sample fragments comprises:
traversing all the basic elements in the reference sample, and when a basic element is comprised in the sample fragment, adding 1 to a first statistic of the basic element.

3. The method according to claim 1 or 2, wherein the dividing the reference sample into at least two reference sub-samples comprises:
traversing all the basic elements in the reference sample, and when a first statistic of a basic element is less than the first threshold, determining that a position of the basic element in the reference sample is a spacing position;
determining the division point according to the spacing position; and
dividing the reference sample into the at least two reference sub-samples according to the division point.

4. The method according to any one of claims 1 to 3, wherein the collecting statistics about a second statistic of each reference sub-sample that is of the reference sample and that comprises the sample fragments comprises:
traversing all the reference sub-samples of the reference sample, and when a reference sub-sample comprises at least one basic element in the sample fragments, adding 1 to a second statistic of the reference sub-sample.

5. The method according to any one of claims 1 to 4, wherein after the combining the adjacent reference sub-samples, when a quantity of the reference sub-samples is greater than a third threshold, the method further comprises:
increasing the second threshold; and
when a sum of second statistics of any adjacent reference sub-samples is less than the increased second threshold, combining the adjacent reference sub-samples.

6. The method according to any one of claims 1 to 5, wherein the data processing system further comprises a second sample set, the second sample set comprises at least two sample fragments, and before the traversing all the sample fragments in the first sample set and collecting statistics about a first statistic of each basic element that is in the reference sample and that is comprised in the sample fragments, the method further comprises:
segmenting the second sample set into at least two first sample sets.

7. The method according to claim 6, wherein the segmenting the second sample set into at least two first sample sets comprises:
determining a segmentation point for the segmentation, wherein the segmentation point comprises positions, in the reference sample, of a preset quantity of basic elements selected at equal intervals from all the basic elements in the reference sample that are sorted according to the preset order; and
traversing all the sample fragments in the second sample set, and determining, according to a position of the segmentation point and a position that is in the reference sample and that is of the first one of basic elements sorted according to the preset order in the sample fragment, a first sample set to which the sample fragment belongs.

8. The method according to claim 6, wherein the segmenting the second sample set into at least two first sample sets comprises:
obtaining a third sample set, wherein the third sample set is a subset of the second sample set, and the third sample set comprises at least two sample fragments;
traversing all the sample fragments in the third sample set, and determining a position that is in the reference sample and that is of the first one of basic elements sorted according to the preset order in the sample fragment;
determining a segmentation point for the segmentation, wherein the segmentation point comprises a preset quantity of positions that are selected at equal intervals from determined positions according to the preset order; and
traversing all the sample fragments in the second sample set, and determining, according to a position of the segmentation point and the position that is in the reference sample and that is of the first one of the basic elements sorted according to the preset order in the sample fragment, a first sample set to which the sample fragment belongs.

9. The method according to claim 7 or 8, wherein the division point further comprises the segmentation point, and before the combining, when a sum of second statistics of any adjacent reference sub-samples is less than a second threshold, the adjacent reference sub-samples, the method further comprises:
combining two reference sub-samples adjacent to the segmentation point when a first statistic of a basic element on the segmentation point is greater than the first threshold.

10. The method according to any one of claims 1 to 9, wherein after the combining, when a sum of second statistics of any adjacent reference sub-samples is less than a second threshold, the adjacent reference sub-samples, the method further comprises:
determining a test sample set, wherein the test sample set comprises sample fragments that are comprised in a same reference sub-sample; and
performing subsequent data processing by using the test sample set as a basic processing unit.

11. The method according to any one of claims 1 to 9, wherein after the dividing the reference sample into at least two reference sub-samples, the method further comprises:
determining a test sample set, wherein the test sample set comprises sample fragments that are comprised in a same reference sub-sample.

12. The method according to claim 11, wherein after the combining the adjacent reference sub-samples, the method further comprises:
combining adjacent test sample sets, wherein a test sample set obtained after the combination comprises a sample fragment that is comprised in a reference sub-sample obtained after the combination; and
performing subsequent data processing by using the test sample set as a basic processing unit.

13. The method according to any one of claims 1 to 12, wherein the basic element comprises nitrogenous base data of deoxyribonucleic acid.

14. The method according to any one of claims 1 to 13, wherein the reference sample comprises reference sequence data of deoxyribonucleic acid.

15. A data processing apparatus, applied to a data processing system, wherein the data processing system comprises a reference sample and a first sample set, the reference sample comprises at least two basic elements sorted according to preset order, the first sample set comprises at least one sample fragment, and the sample fragment comprises at least one basic element captured from the reference sample; and the apparatus comprises:
a first statistics collection module, configured to: traverse all the sample fragments in the first sample set, collect statistics about a first statistic of each basic element that is in the reference sample and that is comprised in the sample fragments, and determine that a position, in the reference sample, of a basic element whose first statistic is less than a first threshold is a spacing position;
a first division module, configured to divide the reference sample into at least two reference sub-samples, wherein a division point for the division comprises: a spacing position that is not adjacent to another spacing position, and any one of at least two adjacent spacing positions;
a second statistics collection module, configured to: traverse all the sample fragments in the first sample set, and collect statistics about a second statistic of each reference sub-sample that is of the reference sample and that comprises the sample fragments; and
a first combination module, configured to combine, when a sum of second statistics of any adjacent reference sub-samples is less than a second threshold, the adjacent reference sub-samples.

16. The apparatus according to claim 15, wherein the first statistics collection module is specifically configured to:
traverse all the basic elements in the reference sample, and when a basic element is comprised in the sample fragment, add 1 to a first statistic of the basic element.

17. The apparatus according to claim 15 or 16, wherein the first division module is specifically configured to:
traverse all the basic elements in the reference sample, and when a first statistic of a basic element is less than the first threshold, determine that a position of the basic element in the reference sample is a spacing position;
determine the division point according to the spacing position; and
divide the reference sample into the at least two reference sub-samples according to the division point.

18. The apparatus according to any one of claims 15 to 17, wherein the second statistics collection module is specifically configured to:
traverse all the reference sub-samples of the reference sample, and when a reference sub-sample comprises at least one basic element in the sample fragments, add 1 to a second statistic of the reference sub-sample.

19. The apparatus according to any one of claims 15 to 18, wherein when a quantity of the reference sub-samples is greater than a third threshold, the first combination module is further configured to:
increase the second threshold; and
when a sum of second statistics of any adjacent reference sub-samples is less than the increased second threshold, combine the adjacent reference sub-samples.

20. The apparatus according to any one of claims 15 to 19, wherein the data processing system further comprises a second sample set, the second sample set comprises at least two sample fragments, and the apparatus further comprises a second division module, configured to:
segment the second sample set into at least two first sample sets.

21. The apparatus according to claim 20, wherein the second division module is specifically configured to:
determine a segmentation point for the segmentation, wherein the segmentation point comprises positions, in the reference sample, of a preset quantity of basic elements selected at equal intervals from all the basic elements in the reference sample that are sorted according to the preset order; and
traverse all the sample fragments in the second sample set, and determine, according to a position of the segmentation point and the position that is in the reference sample and that is of the first one of basic elements sorted according to the preset order in the sample fragment, a first sample set to which the sample fragment belongs.

22. The apparatus according to claim 20, wherein the second division module is specifically configured to:
obtain a third sample set, wherein the third sample set is a subset of the second sample set, and the third sample set comprises at least two sample fragments;
traverse all the sample fragments in the third sample set, and determine a position that is in the reference sample and that is of the first one of basic elements sorted according to the preset order in the sample fragment;
determine a segmentation point for the segmentation, wherein the segmentation point comprises a preset quantity of positions that are selected at equal intervals from determined positions according to the preset order; and
traverse all the sample fragments in the second sample set, and determine, according to a position of the segmentation point and the position that is in the reference sample and that is of the first one of the basic elements sorted according to the preset order in the sample fragment, a first sample set to which the sample fragment belongs.

23. The apparatus according to claim 21 or 22, wherein the division point further comprises the segmentation point, and the apparatus further comprises a second combination module, configured to:
combine two reference sub-samples adjacent to the segmentation point when a first statistic of a basic element on the segmentation point is greater than the first threshold.

24. The apparatus according to any one of claims 15 to 23, wherein the apparatus further comprises a first determining module, configured to:
determine a test sample set, wherein the test sample set comprises sample fragments that are comprised in a same reference sub-sample; and
perform subsequent data processing by using the test sample set as a basic processing unit.

25. The apparatus according to any one of claims 15 to 24, wherein the apparatus further comprises a second determining module, configured to:
determine a test sample set, wherein the test sample set comprises sample fragments that are comprised in a same reference sub-sample.

26. The apparatus according to claim 25, wherein the apparatus further comprises a third combination module, configured to:
combine adjacent test sample sets, wherein a test sample set obtained after the combination comprises a sample fragment that is comprised in a reference sub-sample obtained after the combination; and
perform subsequent data processing by using the test sample set as a basic processing unit.

27. A data processing apparatus, applied to a data processing system, wherein the data processing system comprises a reference sample and a first sample set, the reference sample comprises at least two basic elements sorted according to preset order, the first sample set comprises at least one sample fragment, and the sample fragment comprises at least one basic element captured from the reference sample; and the apparatus comprises:
a processor and a memory coupled to the processor, wherein
the memory is configured to store code; and
the processor is configured to perform the following operations according to the code:
traversing all the sample fragments in the first sample set, collecting statistics about a first statistic of each basic element that is in the reference sample and that is comprised in the sample fragments, and determining that a position, in the reference sample, of a basic element whose first statistic is less than a first threshold is a spacing position;
dividing the reference sample into at least two reference sub-samples, wherein a division point for the division comprises: a spacing position that is not adjacent to another spacing position, and any one of at least two adjacent spacing positions;
traversing all the sample fragments in the first sample set, and collecting statistics about a second statistic of each reference sub-sample that is of the reference sample and that comprises the sample fragments; and
combining, when a sum of second statistics of any adjacent reference sub-samples is less than a second threshold, the adjacent reference sub-samples.
